(19) Europäisches Patentamt
European Patent Office
Office européen des brevets

(11) **EP 3 426 739 B1**

(12) **EUROPEAN PATENT SPECIFICATION**

(45) Date of publication and mention
of the grant of the patent:
**27.05.2020 Bulletin 2020/22**

(21) Application number: **17709998.3**

(22) Date of filing: **13.03.2017**

(51) Int Cl.:
*C09D 167/08* (2006.01)   *C09D 5/16* (2006.01)
*C09D 9/00* (2006.01)   *C02F 1/50* (2006.01)
*A61L 2/232* (2006.01)   *D21H 21/36* (2006.01)

(86) International application number:
**PCT/EP2017/055868**

(87) International publication number:
**WO 2017/153611 (14.09.2017 Gazette 2017/37)**

(54) **USE OF ALKYD RESINS**

VERWENDUNG VON ALKYDHARZEN

UTILISATION DE RÉSINES ALKYDES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.03.2016 EP 16159994**

(43) Date of publication of application:
**16.01.2019 Bulletin 2019/03**

(60) Divisional application:
**20157827.5**

(73) Proprietor: **POM Patentverwaltungs GbR
35619 Braunfels (DE)**

(72) Inventors:
• **SCHEPERS, Klaus
35619 Braunfels (DE)**
• **MISCHO, Horst
54295 Trier (DE)**
• **BIRKEL, Alexander
64287 Darmstadt (DE)**
• **SABANOV, Elena
33129 Delbrueck (DE)**
• **BREMSER, Wolfgang
33102 Paderborn (DE)**

(74) Representative: **Teipel, Stephan et al
Lederer & Keller
Patentanwälte Partnerschaft mbB
Unsöldstrasse 2
80538 München (DE)**

(56) References cited:
EP-A1- 0 344 565   WO-A1-2010/040902
GB-A- 1 595 738   US-A- 3 684 752
US-A- 4 039 494

**Description**

The present invention relates to the use of cured alkyd resins for providing antibacterial properties to at least part of a surface of a substrate.

In many industrial and domestic processes waste water is produced or fresh water is to be stored for later purposes. As essentially all water sources provide non-sterile water, in fresh water and in particular in waste water there is the risk of microbiological contamination during storage or processing.

Many ways are known in the art to avoid creation of microbiological contaminations, in particular biofilms, in containers for the storage of drinking water, waste water, surface water or similar liquids. Further, several methods are known to avoid microbiological contamination of surfaces, e.g. in hospitals or slaughterhouses. For example, usually the surfaces of containers are cleaned by heat treatment, e.g. by rinsing the surfaces or containers with hot liquids or gases, e.g. hot water or steam. Further, treatment by ozone or chlorine containing gases is known for disinfecting surfaces and containers and cleaning and removal of organic contaminations.

It is also known to apply ultraviolet (UV-) radiation to disinfect surfaces, containers, and even liquids. It is also known to add certain substances to the liquids, such as titanium dioxide, or to apply titanium dioxide to a surface for disinfecting or cleaning purposes. However, these surfaces and substances require UV-radiation for activating the disinfecting efficacy of titanium dioxide.

Further, it is known to apply silver ions or copper ions to solutions in order to reduce the microbiological contamination.

All these measures and procedures are disadvantageous as they waste a lot of energy, require high apparatus cost and costs of operation in order to be sufficiently effective, or rather expensive substances, such as silver ions or copper ions, have to be used, the latter having further disadvantages with regard to water and soil pollution. Further, using substances which are dangerous to handle, e.g. ozone or chlorine containing substances, as well as ultraviolet radiation, is disadvantageous and requires additional safety measures.

The use of alkyd resins is known in the art for several purposes, e.g. in synthetic coating and paint industry. However, there was the need, in order to achieve antimicrobial effects in e.g. paints, to add specific antimicrobial agents.

For example US 4,039,494 describes a paint coating to prevent fungal or microbial growths using alkyd resins with an equivalent amount of organotin compound, so that the organotin is chemically incorporated into the resin.

WO 2010/04903 A1 discloses a wood impregnation method, in which an emulsion is absorbed into wood by means of pressure difference. The emulsion contains a reaction product of a fatty acid obtained from a natural oil and a polyalcohol, an emulgator and water. In this way the dimension stability and the water repulsion of wood can be physically improved. It is furthermore said that in addition a good biological durability is achieved. In the examples the resin is prepared in the presence of gum rosin, which is known for its antimicrobial activity. Thus, the emulsion contains a biocidal compound as an internal biocide within the resin. Additionally, the reaction product of a fatty acid with a polyalcohol does not result in an alkyd resin.

GB 1 599 738 discloses a method of producing miscible and water dilutable alkyd coating polymers that are used in paint compositions. It is said that with these compositions microbiological degradability during storage in the paint can be avoided.

There is still a need for further compounds, which exhibit antibacterial activity and which can provide antibacterial properties to at least part of a surface of a substrate.

It has been surprisingly found that alkyd resins alone without any additional external or internal biocides provide excellent antimicrobial efficiency when applied to surfaces, which are in contact with liquids, in particular with waste water, and thereby avoid microbiological contamination of surfaces and of the liquid and lead to a reduction of biofilm formation.

Therefore, the present invention relates to the use of a cured alkyd resin comprising fatty acid residues, which contain at least 6 carbon atoms in the saturated or unsaturated aliphatic chain and no reactive functional group, wherein the cured alkyd resin is free of any biocidal product as defined in Article 3(1)(a) of the Regulation (EU) No. 528-2012 of the European Parliament and the Counsel of 22 May 2012, for providing antibacterial properties to at least part of a surface of a substrate.

Applicants do not wish to be bound to any theory, but it is believed that alkyd resins by themselves exhibit an antimicrobial and in particular antibacterial effect due to physical interaction between the hydrophobic fatty acid side chains of the alkyd polymer and the cell membranes of bacteria contacting a surface equipped with the alkyd resin. The antibacterial efficiency of alkyd resins therefore is based on mere physical or mechanical action.

Said purposes can be characterized by the ability to deter, render harmless, destroy and/or exert a controlling effect on all forms and/or parts of bacteria.

The term "substrate", as referred to herein, includes bodies of any material, including for example polymers, metal, glass and stone, as well as coatings on such bodies. The bodies may have any shape including for example plates, sheets, fibers and tubes.

**EP 3 426 739 B1**

The term "alkyd resin", as used herein, refers to a polyester modified by the addition of fatty acids and/or other components. Alkyd resins are derived from a di-, tri- or polyol and a di-, tri- or polycarboxylic acid or carboxylic acid anhydride. The monomers as such are not encompassed by the term "alkyd resin".

The di-, tri- or polyacid can for example be selected from the group consisting of oxalic acid, malonic acid, succinic acid, glutaric acid, adipic acid, pimelic acid, suberic acid, azelaic acid, cebacic acid, maleic acid, fumaric acid, glutaconic acid, malic acid, aspartic acid, glutamic acid, tartaric acid, phthalic acid, isophthalic acid, terephthalic acid and/or mixtures thereof. Anhydrides of these acids are also suitable. Preferably, the acid residue in the polyester is a diacid residue.

The di-, tri- or polyalcohol can for example be selected from the group consisting of ethylene glycol, butylene glycol, pentanediol, neopentyl glycol, hexylene glycol, diethylene glycol, dipropylene glycol, triethylene glycol, glycerol, trimethylolethane, trimethylopropane, pentaerythritol, methylglucoside, sugars, sugar alcohols, such as mannitol, xylitol and sorbitol and/or mixtures thereof.

The polyester backbone in the alkyd resin is modified by a hydrophobic residue which does not contain any additional reactive groups as defined below. The polyester is modified with a fatty acid residue.

The term "fatty acid" refers to a carboxylic acid having an aliphatic chain. In one embodiment, the aliphatic chain is saturated. In another embodiment, the aliphatic chain is unsaturated. The number of C-atoms in the aliphatic chain is at least 6, such as at least 10 or at least 15.

More preferably the number of C-atoms in the aliphatic chain is in the range of 6 to 40, 6 to 26, 6 to 20, 10 to 40, 10 to 26, 10 to 20, 15 to 40, 15 to 26 or 15 to 20; 15 to 20 being particularly preferred.

Preferably, the fatty acid is selected from the group consisting of caproic acid, caprylic acid, capric acid, myristoleic acid, palmitoleic acid, sapienic acid, oleic acid, elaidic acid, vaccenic acid, linoleic acid, linoelaidic acid, $\alpha$-linolenic acid, arachidonic acid, palmitic acid, stearic acid, lauric acid, myristic acid and/or combinations thereof.

In one embodiment, the alkyd resin is a linear polyester. A linear polyester, as defined herein is a difunctional molecule and has two reactive sites.

In another embodiment, the alkyd resin is a branched polyester containing fatty-acid side groups. A branched polyester, as defined herein is a tri-, tetra-, or higher functional molecule and has three, four, or more reactive sites. Branched polyester may form, as linear polyester, molecular chains, but, in addition, branching connections are formed, which result in a three-dimensional network (cross-linking).

In one embodiment, the alkyd resin is further modified with an unsaturated or saturated oil or the alkyl resin contains such oil.

Preferably, the unsaturated or saturated oil is selected from the group consisting of tung oil, linseed oil, sunflower oil, safflower oil, walnut oil, soybean oil, fish oil, corn oil, tall oil, dehydrated castor oil, coconut oil, rapeseed oil, peppermint oil, lavender oil, safflower oil, walnut oil, fish oil, corn oil, tall oil, dehydrated castor oil, cumin oil, flax oil, vernonia oil and/or mixtures thereof. Linseed oil, sunflower oil, coconut oil, rapeseed oil, peppermint oil, lavender oil and soybean oil, safflower oil, walnut oil, fish oil, corn oil, tall oil, dehydrated castor oil, cumin oil, flax oil and vernonia oil being particularly preferred.

The above oils can be present in the alkyd resin as such or as derivatives thereof, for example as their fatty acid, monoester, fatty acid diester, and esters with other carboxylic acids, such as ascorbic acid, acetic acid, propionic acid, benzoic acid or lactic acid.

Further possible additives in the alkyd resin are for example fatty acids, in particular those as described above, oct-1-en-3-ol, citronellal and pheromones of clothing mots, such as for example (Z, E)-tetradeca-9,12-dienyl acetate.

Alkyd resins comprising an unsaturated or saturated oil are classified by the amount of oil based on the total weight of the resin. In one embodiment, the amount of unsaturated or saturated oil is higher than 60 wt.-% (long-oil alkyd resin), between 40 wt.-% and 60 wt.-% (medium-oil alkyd resin), or lower than 40 wt.-% (short-oil alkyd resin) based on the total weight of the alkyd resin. In a preferred embodiment the alkyd resin is a medium-oil alkyd resin or a long-oil alkyd resin.

In one embodiment the alkyd resin is WorléeKyd B 845 (Worlée-Chemie GmbH, Germany), Setal 196 XX (Nuplex, USA, Kentucky) or Alkydal F26 XX, Alkydal F251 X (Covestro, Germany).

In one embodiment, the alkyd resin additionally reduces the growth of a biofilm on the surface of the substrate. The term "additionally" as used herein has the meaning of "simultaneously" and/or "sequentially".

The term "biofilm", as used herein, refers to an assembly of microorganisms wherein cells stick to each other on the surface of a substrate. The term "growth of biofilm" or "biofilm growth", as used herein, refers to the microorganism built-up adhering to a surface of a substrate.

The term "reduction of the growth of a biofilm", as used herein, refers to the decrease, preferably the inhibition, of the microorganism built-up on a long term scale, i.e. at least 1 week, preferably at least 1 month, more preferably at least 1 year, even more preferably at least 3 years, on the surface of a substrate comprising an alkyd resin as described herein compared to the surface of a substrate not comprising an alkyd resin as described herein.

In one embodiment, the alkyd resins as described herein are particularly suitable for simultaneously imparting antibacterial properties to a surface of a substrate and reducing, particularly inhibiting, the growth of a biofilm on the surface of the substrate, thereby reducing, preferably inhibiting, the growth of biofilm on a substrate surface and maintaining the antibacterial efficacy of the alkyd resins on a long term time scale (at least 2 weeks, preferably at least 1 month, more preferably at least 1 year, even more preferably at least 3 years).

In one embodiment, the alkyd resin is a drying or a non-drying alkyd resin.

Drying alkyd resins cure at approximately 20°C or by drying at 60°-80°C. Non-drying alkyd resins do not dry at these temperatures, but are cured by baking.

In one embodiment, the alkyd resin further comprises a siccative (oil-drying agent), which catalyzes the curing of the alkyd resins. Siccatives are typically derived from cobalt, manganese and iron ions.

In another embodiment, the alkyd resin does not contain any siccatives.

A particular advantage of the use, according to the present invention, is the finding that alkyd resins itself without the requirement of any further biocidally acting compound provides the desired antibacterial effect. Therefore, in the present invention the alkyd resin is free of any biocidal product as defined in Article 3(1)(a) of the Regulation (EU) No. 528-2012 of the European Parliament and the Counsel of 22 May 2012. In particular, the alky resin used in the present invention does not contain any nanoparticles, such as metal nanoparticles, in particular silver nanoparticles. The alkyd resin is free of silver, organotin and gum rosin.

In further embodiment the alkyd resin used according to the present invention does not contain any internal biocides, preferably the alkyd resin does not contain any internal biocides and any external biocides. In this context "internal biocides" are understood as compounds having biocidal activity (in particular those compounds as defined in Article 3(1)(a) of the Regulation (EU) No. 528-2012 of the European Parliament and the Counsel of 22 May 2012), which are chemically bound to the alkyd resin, such as the gum rosin in the alkyd resin of example 1 of WO 2010/040903 or the organotin compound in the alkyd resin of US 4,039,494. "External biocides" are to be understood as those biocidal compounds which are present in the alkyd resin, but which are not chemically bound to the alkyd polymer.

Since it is believed that the antibacterial effect of the alkyd resin is due to a mechanical interaction between the hydrophobe fatty acid residue side chains of the polyester polymer and the cell membranes of bacteria, it is desirable that the fatty acid residues have a certain hydrophobicity but do not react with the cell membranes. Therefore, the fatty acid residues in the alkyd resins do not contain any reactive functional groups, such as hydroxy, amino, sulfo, phosphato, carboxy, carboxy-amid, cyano, etc. Possible unsaturations in the fatty acid residues are not considered as reactive functional groups in the sense of the present invention.

The above described qualities can be used to modify surfaces of substrates. Wherein one or more parts (e.g. materials, additives, surface layers, inner layers, exterior, interior, etc.) of the said substrate comprise an alkyd resin as described herein, and/or one or more of the surfaces (e.g. functional surfaces) of the said substrate are at least partially coated with surface layers, paints or coatings, wherein said surface layers, paints or coatings comprise an alkyd resin.

The substrates can be selected from the group consisting of: a polymer body, a paint, a varnish, a coating, an ink, glass fibers and an inorganic oxide material, containers for storage of drinking water (e.g. in the beverage industry), waste water, or surface water; containers for waste decomposition; containers for water purification; hospitals, medical equipment and devices, wound dressings, diapers, household appliances, water boilers, heating systems, slaughterhouses, ships, boats, roof coverings, roof tiles, indoor tiles, outdoor tiles, kitchen rooms, sinks, bathroom equipment, wash closets, toilets, portable toilets, ceramics, polymers, fibers, rain water sewers, exterior façades, elements of façades, pools, pumps, tubing, walls, floorings, laminates, technical textiles, activewear fabrics, textile fibres, paper, wood, apparatus for air and water purification, apparatus for soil decontamination, window glass (e.g. self-cleaning windows), mirrors (e.g. anti-fog coatings for mirrors and/or glass), filter materials such as nonwovens, respirator masks, air filters, such as air-conditioner filters, water filters, and activated-carbon filters for water, air purification, and/or (food) packaging.

In one embodiment of the present invention the alkyd resin is further combined with a polymer such as a thermoplastic polymer, an elastomer, a thermoplastic elastomer, a duroplast or a mixture thereof.

The term "thermoplastic", as used herein, refers to a polymer that becomes pliable or moldable above a specific temperature and solidifies upon cooling. Examples for thermoplastic polymers include but are not limited to polyacrylates, acrylonitrile-butadiene-styrenes, polyamides such as nylon, polyacetic acid, polybenzimidazole, polycarbonate, polyether sulfone, polyetherether ketone, polyetherimide, polyethylene, polyphenylene oxide, polyphenylen sulfide, polypropylene, polystyrene, polyvinylchloriode, polyethyleneterephthalate, polyurethane, polyester and polytetrafluoroethylene (e.g. Teflon).

The term "elastomer", as used herein, refers to a polymer with viscoelasticity (having both viscosity and elasticity). Examples for elastomers include but are not limited to unsaturated rubbers such as natural polyisoprene (natural rubber), synthetic polyisoprene, polybutadiene, chloroprene rubber, butyl rubber, styrene-butadiene rubber, (hydrogenated) nitrile rubber, saturated rubbers such as ethylene propylene rubber, ethylene propylene diene rubber, epichlorohydrin rubber, polyacrylic rubber, silicone, silicone rubber, fluorosilicone rubber, fluoro- and perfluoroelastomers, and ethylene-vinyl acetate.

The term "thermoplastic elastomer", as used herein, refers to a class of copolymers or a physical mix of polymers which consists of materials with both thermoplastic and elastomeric properties. Examples for thermoplastic elastomers include but are not limited to styrenic block copolymers, polyolefin blends, elastomeric alloys, thermoplastic polyurethanes, thermoplastic copolyester, and thermoplastic polyamides.

The term "duroplast", as used herein, refers to a polymer which is no longer pliable after curing. Examples for duroplasts include but are not limited to aminoplasts, phenoplasts, epoxy resins, polyacrylates, polyurethanes, polyesters, urea formaldehyde resins, melamine formaldehyde resins, and phenol formaldehyde resins.

In a more preferred embodiment of the present invention, the substrate comprises a polymer selected from the group consisting of polypropylene, polyethylene, polyethyleneterephthalate, polyester, polyamide, polyurethane, polyacrylate, polycarbonate, polystyrene, polyimides, polymethacrylates, polyoxoalkylenes, poly(phenylene oxides), polyvinylesters, polyvinylethers, polyvinylidene chloride, acrylonitrile-butadiene-styrene, natural and synthetic polyisoprene, polybutadiene, chloroprene rubber, styrene-butadiene rubber, tetrafluoroethylene, silicone, acrylate resins, polyurethane resins, silicone resins, polyester resins, alkyd resins, epoxy resins, phenolic resins, and urea or amine based resins, or a mixture thereof.

Further examples of polymers are polyolefins, such as polyethylene and polypropylene, or melamine resins (melamine-formaldehyde resins). Melamine-formaldehyde resins form via the condensation of formaldehyde with melamine. For example, the amount of the alkyd resin is in the range of 10 to 90 wt.-% and the amount of the melamine resin is in the range of 10 to 90 wt.-% based on the total weight of the two resins. In a preferred embodiment, the alkyd resin is in the range of 50 to 90 wt.-% and the amount of the melamine resin is in the range of 10 to 50 wt.-% based on the total weight of the two resins. Particularly preferred, the alkyd resin is about 70 wt.-% and the amount of the melamine resin is about 30 wt.-% based on the total weight of the two resins. An example of a suitable melamine resin is Maprenal MF 900 (INEOS Melamines GmbH, Germany). It was found that the combination of an alkyd resin with a melamine resin improves surface hardness and scratch resistance and reduces curing time.

The incorporation of the alkyd resin into a polymer body can e.g. be achieved by mixing the alkyd resin with the polymer in order to obtain a dispersion or a compound of alkyd resin and polymer. Said dispersion or compound can then e.g. be submitted to injection molding or extrusion for forming the polymer body.

Alternatively, the single components of the alkyd resin can be mixed with the polymers before polymerization of the alkyd resin is carried out.

The resulting polymer can e.g. be submitted to compounding processes, pressureless processing techniques (e.g. casting, dipping, coating, foaming) or compression molding, rolling and calendaring, extrusion, blow molding or injection molding processes or drawing, thermoforming or printing for forming the polymer body.

The polymer body as described herein can also be obtained by drylaid, airlaid, spunlaid/meltblown or wetlaid processes, in particular if the polymer body is for use as fiber material or non-woven material.

The abbreviation "wt.-%" or "w/w", as used herein, means "weight percentage" and refers to the weight amount of a compound in relation to the (total) weight of a composition of compounds or of a substrate if nothing else is explicitly stated or obvious under the circumstances.

**Examples**

The value of the antibacterial activity (R-value) is calculated as follows:

$$R = \log (B/A) - \log (C/A)$$

| A | mean value of colony-forming unit (CFU) of untreated surfaces, directly after application (0 h) |
|---|---|
| B | mean value of colony-forming unit (CFU) of untreated surfaces, after incubation (24 h, 36 °C, 90% relative humidity (rH)) |
| C | mean value of mean colony-forming unit (CFU) of treated surfaces, after incubation (24 h, 36 °C, 90% relative humidity (rH)) |

**Example 1**

Alkyd resins and combinations of alkyd resin with melamine resins were tested on their antibacterial effect.

The antibacterial tests were performed on Polypropylen (PP) - plates having a size of 15 cm². The area of the blind value testing was 16 cm². Therefore, the values of the antibacterial testing were extrapolated to 16 cm². The bacterium tested for was the gram-positive bacterium Staphylococcus aureus (DSM 799).

EP 3 426 739 B1

The results are summarized in the following table 1:

Table 1

| Sample | CFU / cm$^2$ [mean of three tests] | | R-value |
| --- | --- | --- | --- |
| | after 0 hours | after 24 hours | |
| blank value | $6.0 \times 10^7$ | $1.5 \times 10^8$ | --- |
| WorleeKyd B 845 | --- | < 4 | > 7.66 |
| Alkydal F 26 X | --- | < 4-50 | > 7.14 - 5.87 |
| Alkydal F 251 X / Maprenal MF 900 (70 wt.-%/30 wt.-%) | --- | < 4 | > 6.87 |

It can be seen from the above table that all of the tested alkyd resins as well as the combination of alkyd resin with melamine resin show excellent antibacterial effects.

Example 2

The test as described in example 1 was repeated. Prior to testing, the resins were washed with demineralized water or tap water over 24 hours.

The results are summarized in the following table 2:

Table 2

| Sample | CFU / cm$^2$ [mean of three tests] | | R-value |
| --- | --- | --- | --- |
| | after 0 hours | after 24 hours | |
| blank value | $1.6 \times 10^7$ | $4.5 \times 10^7$ | --- |
| WorleeKyd B 845 washed with demineralized water | --- | < 5000 | > 3.95 |
| WorleeKyd B 845 washed with tap water | --- | < 5000 | > 3.95 |
| WorleeKyd B 845 w/o siccative washed with demineralized water | --- | < 5000 | > 3.95 |
| WorleeKyd B 845 w/o siccative washed with tap water | --- | < 5000 | > 3.95 |
| Setal 196 XX washed with demineralized water | --- | < 5000 | > 3.95 |
| Setal 196 XX washed with demineralized water | --- | < 5000 | > 3.95 |
| Demineralized water: <1 µS conductivity | | | |

It can be seen from the above table that all of the tested alkyd resins show excellent antibacterial effects even after washing over 24 hours in demineralized or tap water.

Example 3

The test as described in example 1 was repeated. Prior to testing, the resins were eluted 3 weeks in 100 ml tap water.

The results are summarized in the following table 3:

Table 3

| Sample | CFU / cm$^2$ | | R-value |
| --- | --- | --- | --- |
| | after 0 hours | after 24 hours | |
| blank value | $5.4 \times 10^7$ | $1.1 \times 10^8$ | --- |
| WorleeKyd B 845 without siccative | --- | 680 | 5.19 |
| Setal 196 XX | --- | $2.8 \times 10^5$ | 2.58 |

EP 3 426 739 B1

It can be seen from the above table that all of the tested alkyd resins show excellent antibacterial effects even after elution in water over 3 weeks.

**Example 4**

The test as described in example 1 was repeated, with the modification that the gram-negative bacterium Escherichia coli (DSM 1116) was used as a test microorganism.
The results are summarized in table 4 below:

Table 4

| Sample | CFU / cm$^2$ [mean of three tests] | | R-value |
|---|---|---|---|
| | after 0 hours | after 24 hours | |
| blank value | $2.4 \times 10^7$ | $2.2 \times 10^8$ | --- |
| Alkydal F 26 X | --- | < 3 | > 6.93 |
| WorleeKyd B 845 | --- | < 3 | > 6.93 |
| Setal 196 XX | --- | < 3 | > 6.93 |

As can be seen from the table above, also against the gram-negative Escherichia coli, the different alkyd resins display high antibacterial effectiveness; comparable in extent to what was observed for the gram-positive Staphylococcus aureus.

**Example 5**

In order to exclude that the antibacterial effect was based on acidic pH of the resins, the pH was measured prior and after washing with a few milliliters of demineralized water and tap water.
The results are summarized in the following table 5:

Table 5

| | | pH | | |
|---|---|---|---|---|
| | water | 0 h | 24 h | 72 h |
| WorleeKyd B 845 | demineralized | 5.56 | 4.83 | 5.41 |
| | tap | 7.49 | 7.81 | 7.96 |
| WorleeKyd B 845 without siccative | demineralized | 5.56 | 4.84 | 5.48 |
| | tap | 7.49 | 7.88 | 7.79 |
| Setal 196 XX | demineralized | 5.56 | 5.24 | 5.37 |
| | tap | 7.49 | 8.33 | 8.20 |

It can be seen from the above table that the pH of the tested alkyd resins does not alter significantly and thus it can be followed that the antibacterial effect of the alkyd resins is not based on acidic pH.

**Example 6**

The alkyd resins and combination of alkyd resin with melamine resin were tested for their mechanical properties.
The results are summarized in the following table 6:

Table 6

| | Cross hatch test according to DIN EN ISO 2409 [adhesion] | Pencil hardness [scratch resistance] | Indentation hardness [mechanical strength] |
|---|---|---|---|
| WorleeKyd B845 | 0/0 | 6B | 47.6 |

(continued)

| | Cross hatch test according to DIN EN ISO 2409 [adhesion] | Pencil hardness [scratch resistance] | Indentation hardness [mechanical strength] |
|---|---|---|---|
| Alkydal F 251 X / Maprenal MF 900 (70 wt.-%/30 wt.-%) | 0/1 | F | 83.3 |

It can be seen from the above table that all of the tested alkyd resins as well as the combination of alkyd resin with melamine resin show excellent mechanical properties. The combination of 70 wt.-% alkyd resin with 30 wt.-% melamine resin even further improved adhesion, scratch resistance and mechanical strength of the resin.

**Example 7**

Alkyd resins and combination of alkyd resin with melamine resins were tested for their effectiveness against biofilm formation. For that, the test samples, as described in example 1, were added to individual wells of a multi-well plate. Biofilm medium, inoculated with Staphylococcus aureus at $10^6$ CFU/ml, was added to individual wells. The samples were removed after different times, rinsed with sterile water and dried. The dried samples were examined using scanning electron microscopy.

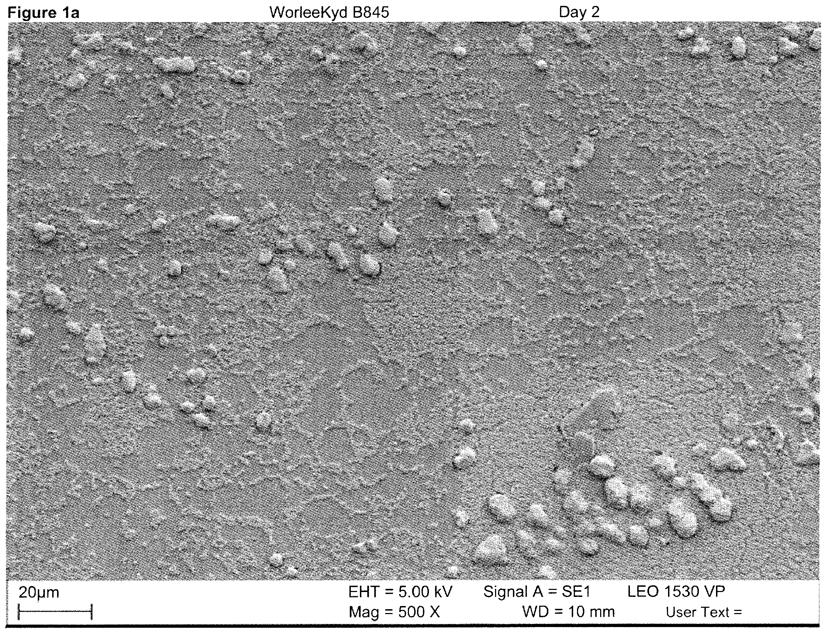

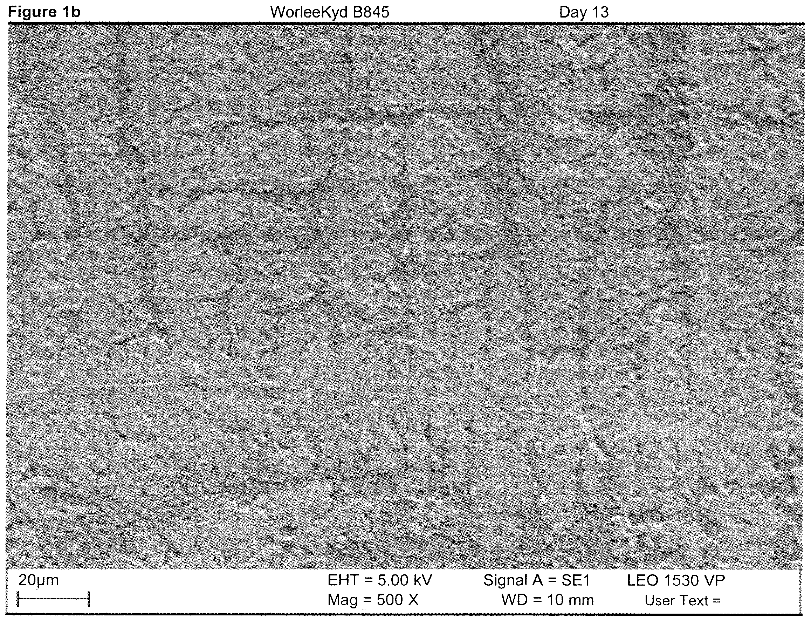

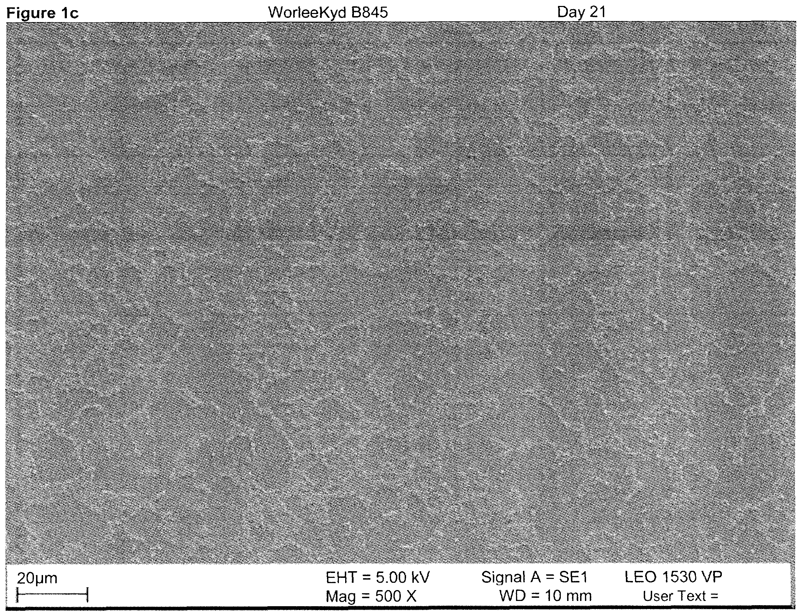

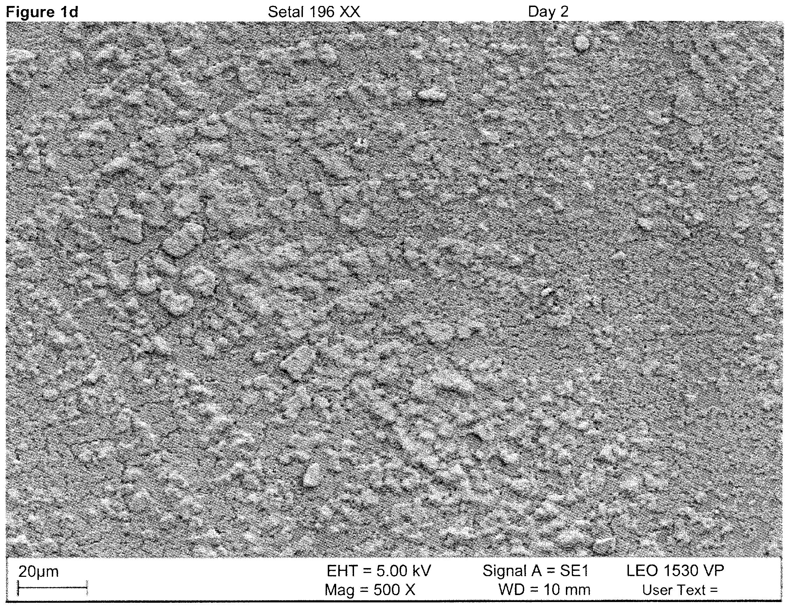

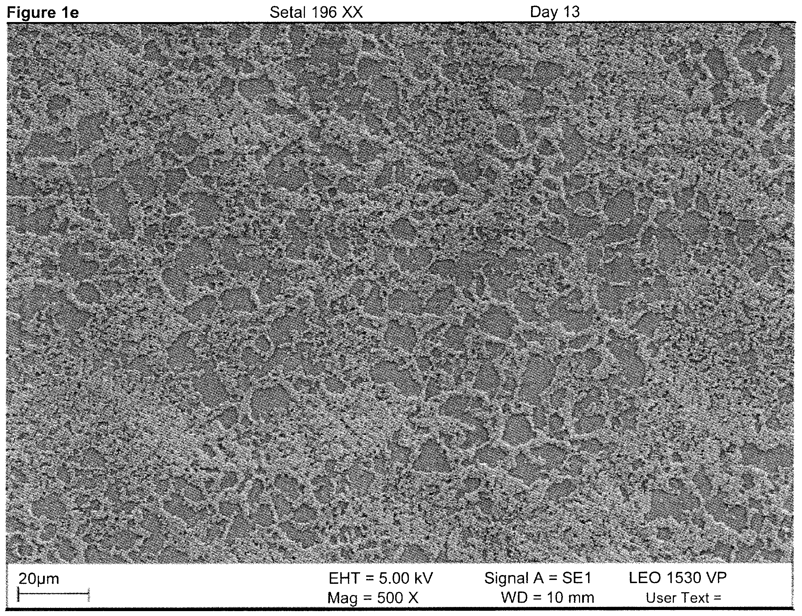

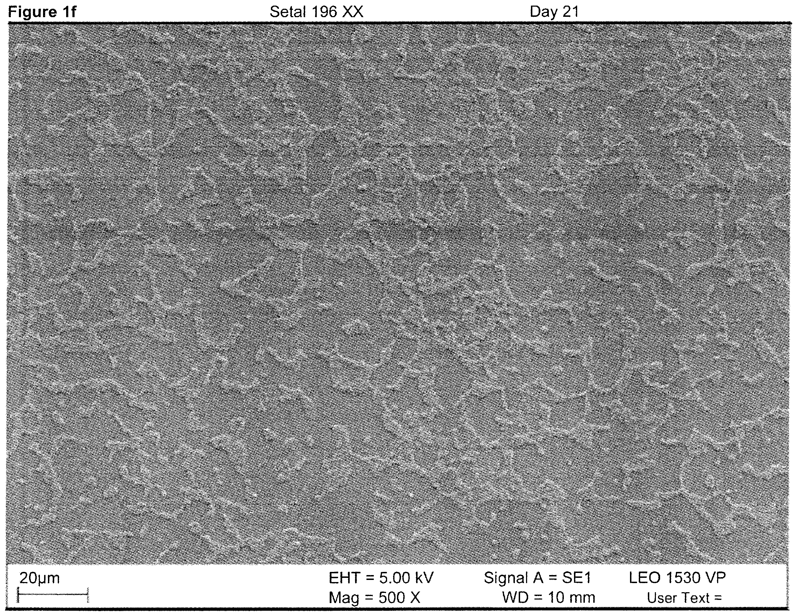

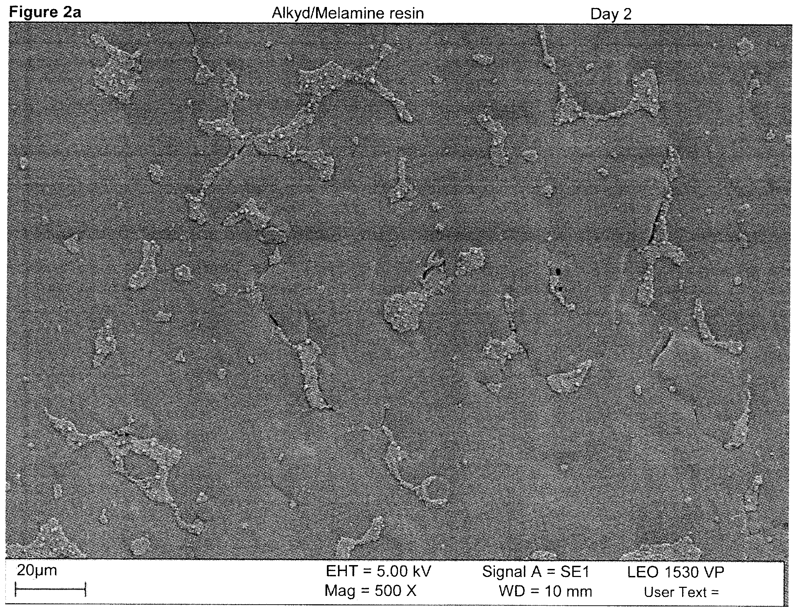

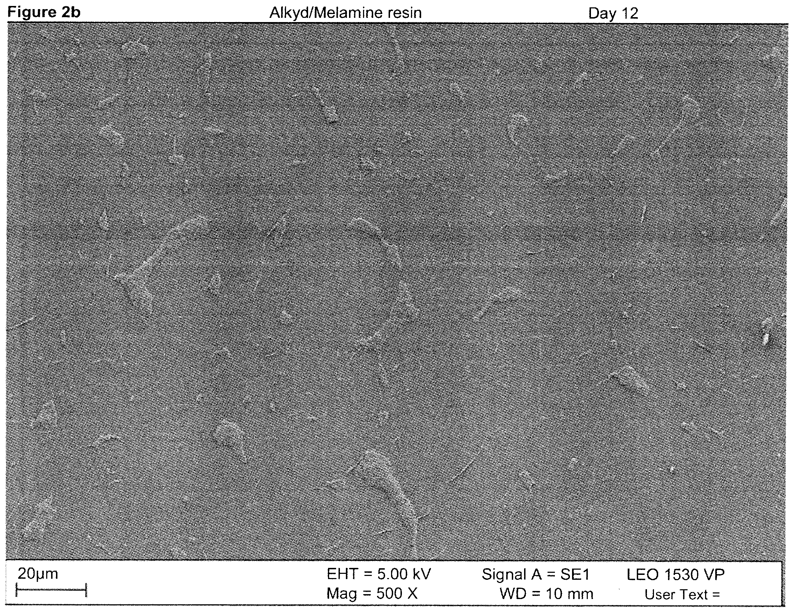

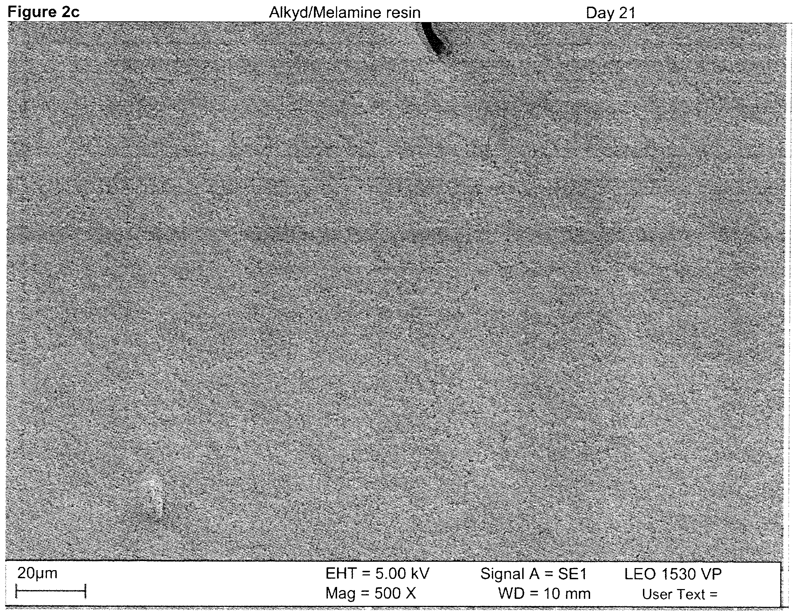

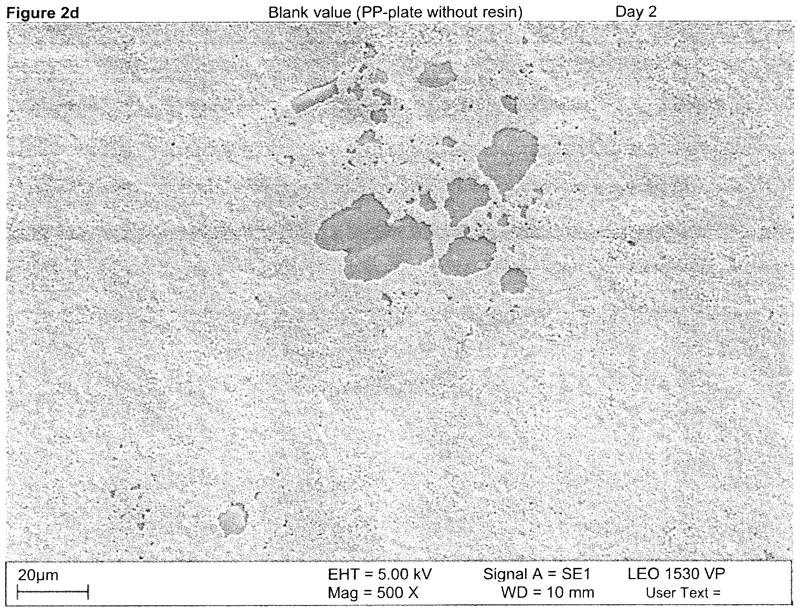

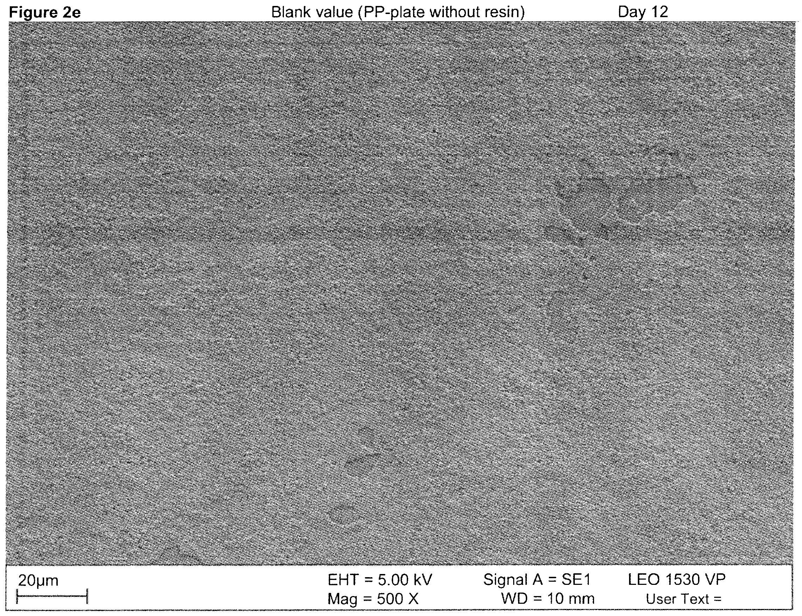

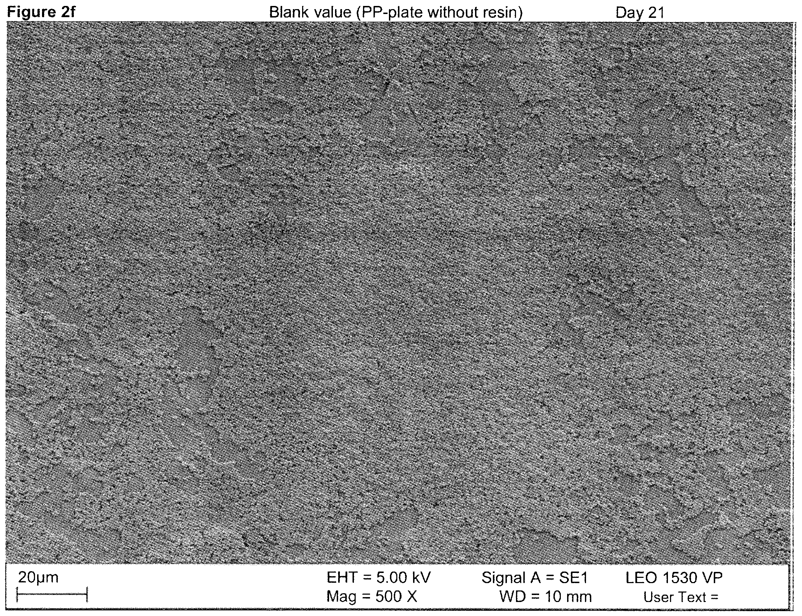

As can be seen from the images in Figures 1 and 2, for the alkyd resins after 21 days there are large gaps in the biofilm. For the alkyd resin with melamine resin, biofilm formation after 12 days is strongly reduced. This demonstrates that both alkyd resins and alkyd resins with melamine resins lead to a strong reduction in biofilm formation.

**Example 8**

The test as described in example 1 was repeated. Two alkyd resins obtained from Worlee-Chemie GmbH were tested, namely WorleeKyd B 845 and Worleesol E 330 (WSE 330). In order to exclude the presence of any low molecular weight compounds in the alkyd resin coatings, these were extracted for 24 hours in water, one hour in white spirit and one minute in butanol, respectively. For butanol the time of extraction was short because butanol is an excellent solvent also for the alkyd resin itself and it was found that the coating itself was destroyed after 2 minutes. Therefore an extraction time of only one minute was selected.

Extracting the alkyd resins with the solvents of different polarity ensures that the coating do not contain any small molecular weight residues which could influence the antibacterial activity of the coating. The observed activities are therefore exclusively based on the alkyd resin itself.

The results are summarized in the following table 7:

Table 7

| Sample | CFU / cm$^2$ [mean of three tests] | | R-value |
|---|---|---|---|
| | after 0 hours | after 24 hours | |
| WorleeKyd B 845 + Siccative (blind) | --- | < 4 | > 7.15 |
| WorleeKyd B 845 + Sicc. + H$_2$O 24h | --- | < 4 | > 7.15 |
| WorleeKyd B 845 + Sicc. + white spirit 60 min | --- | < 4 | > 7.15 |
| WorleeKyd B 845 + Sicc. + butanol 1 min | --- | < 4 | > 7.15 |
| WSE 330 (blind) | --- | < 4 | > 7.15 |
| WSE 330 + H$_2$O 24h | --- | < 4 | > 7.15 |
| WSE 330 + white spirit 60 min. | --- | < 4 | > 7.15 |
| WSE 330 + butanol 1 min | --- | < 4 | > 7.15 |
| blank value | 1.5 x 10$^7$ | 4.8 x 10$^8$ | --- |

It can be seen from the data in the above table that those samples which were extracted with water, white spirit and butanol, respectively, do not exhibit a higher antibacterial activity compared to the alkyd resin coating without extraction (blind). It is therefore evident that the observed antibacterial activity is due to the alkyd resin as such and not due to the presence of any low molecular weight molecules.

**Example 9**

Two alkyd resins obtained from Worlee-Chemie GmbH were tested, namely WorleeKyd B 845 and Worleesol E 330 (WSE 330). After polymerization of the alkyd resins, the resins have been milled and used as an additive for polypropylene in an injection moulding process.

The results are summarized in the following table 8:

Table 8

| Sample | CFU / cm$^2$ [mean of three tests] | | R-value |
| --- | --- | --- | --- |
| | after 0 hours | after 24 hours | |
| WSE 330 before hardening (loading 8,4 % dry substance) in polypropylene | --- | 3.0 x 10$^5$ | 1.16 |
| WSE 330 after hardening and milling (loading 10%) in polypropylene | --- | 1.6 x 10$^5$ | 1.42 |
| WorleeKyd B 845 + Sicc. after hardening without milling (loading 10%) in polypropylene | --- | 4.0 x 10$^4$ | 2.03 |
| blank value | 2.4 x 10$^6$ | 4.3 x 10$^6$ | --- |

It can be seen from the data in the above table that alkyd resins can be incorporated in polyolefins as an additive by an injection moulding process in order to produce polyolefins with an antibacterial functionality.

**Claims**

1. Use of a cured alkyd resin comprising fatty acid residues, which contain at least 6 carbon atoms in the saturated or unsaturated aliphatic chain and no reactive functional group, wherein the cured alkyd resin is free of any biocidal product as defined in Article 3(1)(a) of the Regulation (EU) No. 528-2012 of the European Parliament and the Counsel of 22 May 2012, for providing antibacterial properties to at least part of a surface of a substrate.

2. Use of an alkyd resin according to claim 1, wherein the alkyd resin is a polyester modified with fatty acids.

3. Use of an alkyd resin according to claim 1 or 2, wherein the alkyd resin is a linear polyester.

4. Use of an alkyd resin according to claim 1 or 2, wherein the alkyd resin is a branched polyester containing fatty-acid side groups.

5. Use of an alkyd resin according to any one of the preceding claims, wherein the alkyd resin is further modified with or contains an unsaturated or saturated oil.

6. Use of an alkyd resin according to claim 5, wherein the unsaturated or saturated oil is selected from the group consisting of linseed oil, sunflower oil, coconut oil, rapeseed oil, peppermint oil, lavender oil, soybean oil, safflower oil, walnut oil, fish oil, corn oil, tall oil, dehydrated castor oil, cumin oil, flax oil, wood oil, vernonia oil and/or mixtures thereof.

7. Use of an alkyd resin according to claim 5 or 6, wherein the amount of unsaturated or saturated oil is higher than 60 wt.-% (long-oil alkyd resin), between 40 wt.-% and 60 wt.-% (medium-oil alkyd resin), or lower than 40 wt.-% (short-oil alkyd resin) based on the total weight of the alkyd resin.

8. Use of an alkyd resin according to claim 7, wherein the alkyd resin is a medium-oil alkyd resin or a long-oil alkyd resin.

9. Use of an alkyd resin according to any one of the preceding claims, wherein the alkyd resin is a drying or non-drying alkyd resin.

10. Use of an alkyd resin according to any one of the preceding claims, wherein the alkyd resin further comprises a siccative.

11. Use of an alkyd resin according to any one of the preceding claims, wherein the substrate is selected from the group consisting of a polymer body, a paint, a varnish, a coating, an ink, glass fibers and/or an inorganic oxide material.

12. Use of an alkyd resin according to any one of the preceding claims, wherein the alkyd resin is further combined with another polymer, such as a thermoplastic polymer, a thermoplastic elastomer, a duroplast or an elastomer.

13. Use of an alkyd resin according to any one of the preceding claims, wherein the alkyd resin additionally reduces the growth of a biofilm on the surface of the substrate.

14. Use of an alkyd resin according to any one of the preceding claims, wherein the alkyd resin is combined with a melamine resin.

**Patentansprüche**

1. Verwendung eines gehärteten Alkydharzes, umfassend Fettsäurereste, die mindestens 6 Kohlenstoffatome in der gesättigten oder ungesättigten aliphatischen Kette und keine reaktive funktionelle Gruppe enthalten, wobei das gehärtete Alkydharz frei von jeglichem biozidem Produkt, wie in Artikel 3(1)(a) der Verordnung (EU) Nr. 528-2012 des Europäischen Parlaments und des Rates vom 22. Mai 2012 definiert, ist, um zumindest einen Teil einer Oberfläche eines Substrats mit antibakteriellen Eigenschaften zu versehen.

2. Verwendung eines Alkydharzes nach Anspruch 1, wobei das Alkydharz ein mit Fettsäuren modifizierter Polyester ist.

3. Verwendung eines Alkydharzes nach Anspruch 1 oder 2, wobei das Alkydharz ein linearer Polyester ist.

4. Verwendung eines Alkydharzes nach Anspruch 1 oder 2, wobei das Alkydharz ein verzweigter Polyester ist, der Fettsäure-Seitengruppen enthält.

5. Verwendung eines Alkydharzes nach einem der vorstehenden Ansprüche, wobei das Alkydharz ferner mit einem ungesättigten oder gesättigten Öl modifiziert ist oder dieses enthält.

6. Verwendung eines Alkydharzes nach Anspruch 5, wobei das ungesättigte oder gesättigte Öl ausgewählt ist aus der Gruppe, bestehend aus Leinöl, Sonnenblumenöl, Kokosöl, Rapsöl, Pfefferminzöl, Lavendelöl, Sojaöl, Safloröl, Walnussöl, Fischöl, Maiskeimöl, Tallöl, dehydratisiertem Rizinusöl, Cuminöl, Flachsöl, Holzöl, Vernoniaöl und/oder Gemischen davon.

7. Verwendung eines Alkydharzes nach Anspruch 5 oder 6, wobei die Menge an ungesättigtem oder gesättigtem Öl mehr als 60 Gew.-% beträgt (langöliges Alkydharz), zwischen 40 Gew.-% und 60 Gew.-% liegt (mittelöliges Alkydharz) oder weniger als 40 Gew.-% beträgt (kurzöliges Alkydharz), basierend auf dem Gesamtgewicht des Alkydharzes.

8. Verwendung eines Alkydharzes nach Anspruch 7, wobei das Alkydharz ein mittelöliges Alkydharz oder ein langöliges Alkydharz ist.

9. Verwendung eines Alkydharzes nach einem der vorstehenden Ansprüche, wobei das Alkydharz ein trocknendes oder nicht-trocknendes Alkydharz ist.

10. Verwendung eines Alkydharzes nach einem der vorstehenden Ansprüche, wobei das Alkydharz ferner ein Sikkativ umfasst.

11. Verwendung eines Alkydharzes nach einem der vorstehenden Ansprüche, wobei das Substrat aus der Gruppe ausgewählt ist, bestehend aus einem Polymerkörper, einer Farbe, einem Lack, einer Beschichtung, einer Tinte, Glasfasern und/oder einem anorganischen Oxidmaterial.

12. Verwendung eines Alkydharzes nach einem der vorstehenden Ansprüche, wobei das Alkydharz ferner mit einem anderen Polymer, wie einem thermoplastischen Polymer, einem thermoplastischen Elastomer, einem Duroplasten oder einem Elastomer, kombiniert wird.

13. Verwendung eines Alkydharzes nach einem der vorstehenden Ansprüche, wobei das Alkydharz zusätzlich das Wachstum eines Biofilms auf der Oberfläche des Substrats verringert.

14. Verwendung eines Alkydharzes nach einem der vorstehenden Ansprüche, wobei das Alkydharz mit einem Melaminharz kombiniert wird.

**Revendications**

1. Utilisation d'une résine alkyde durcie comprenant des résidus d'acides gras, qui contiennent au moins 6 atomes de carbone dans la chaîne aliphatique saturée ou insaturée et aucun groupe fonctionnel réactif, dans laquelle la résine alkyde durcie est exempte de tout produit biocide tel que défini dans l'article 3(1)(a) du Règlement (UE) n° 528-2012 du Parlement européen et du Conseil du 22 mai 2012, pour conférer des propriétés antibactériennes à au moins une partie d'une surface d'un substrat.

2. Utilisation d'une résine alkyde selon la revendication 1, dans laquelle la résine alkyde est un polyester modifié avec des acides gras.

3. Utilisation d'une résine alkyde selon la revendication 1 ou 2, dans laquelle la résine alkyde est un polyester linéaire.

4. Utilisation d'une résine alkyde selon la revendication 1 ou 2, dans laquelle la résine alkyde est un polyester ramifié contenant des groupes latéraux d'acides gras.

5. Utilisation d'une résine alkyde selon l'une quelconque des revendications précédentes, dans laquelle la résine alkyde est en outre modifiée avec ou contient une huile insaturée ou saturée.

6. Utilisation d'une résine alkyde selon la revendication 5, dans laquelle l'huile insaturée ou saturée est choisie dans le groupe constitué par l'huile de graines de lin, l'huile de tournesol, l'huile de noix de coco, l'huile de colza, l'huile de menthe poivrée, l'huile de lavande, l'huile de soja, l'huile de carthame, l'huile de noix, l'huile de poisson, l'huile de maïs, l'huile de pin, l'huile de ricin déshydratée, l'huile de cumin, l'huile de lin, l'huile de bois, l'huile de vernonia et/ou leurs mélanges.

7. Utilisation d'une résine alkyde selon la revendication 5 ou 6, dans laquelle la quantité d'huile insaturée ou saturée est supérieure à 60 % en poids (résine alkyde à huile longue), comprise entre 40 % en poids et 60 % en poids (résine alkyde à huile moyenne), ou inférieure à 40 % en poids (résine alkyde à huile courte) par rapport au poids total de la résine alkyde.

8. Utilisation d'une résine alkyde selon la revendication 7, dans laquelle la résine alkyde est une résine alkyde à huile moyenne ou une résine alkyde à huile longue.

9. Utilisation d'une résine alkyde selon l'une quelconque des revendications précédentes, dans laquelle la résine alkyde est une résine alkyde sèche ou non sèche.

10. Utilisation d'une résine alkyde selon l'une quelconque des revendications précédentes, dans laquelle la résine alkyde comprend en outre un siccatif.

11. Utilisation d'une résine alkyde selon l'une quelconque des revendications précédentes, dans laquelle le substrat est choisi dans le groupe constitué par un corps polymère, une peinture, un vernis, un revêtement, une encre, des fibres de verre et/ou un matériau d'oxyde inorganique.

12. Utilisation d'une résine alkyde selon l'une quelconque des revendications précédentes, dans laquelle la résine alkyde est en outre combinée avec un autre polymère, tel qu'un polymère thermoplastique, un élastomère thermoplastique, une matière thermodurcissable ou un élastomère.

13. Utilisation d'une résine alkyde selon l'une quelconque des revendications précédentes, dans laquelle la résine alkyde réduit en outre la croissance d'un biofilm sur la surface du substrat.

14. Utilisation d'une résine alkyde selon l'une quelconque des revendications précédentes, dans laquelle la résine alkyde est combinée avec une résine de mélamine.

**Figure 1a**   WorleeKyd B845   Day 2

20µm
EHT = 5.00 kV   Signal A = SE1   LEO 1530 VP
Mag = 500 X   WD = 10 mm   User Text =

Figure 1b

**Figure 1c**          WorleeKyd B845                    Day 21

| 20µm | EHT = 5.00 kV | Signal A = SE1 | LEO 1530 VP |
| | Mag = 500 X | WD = 10 mm | User Text = |

EP 3 426 739 B1

**Figure 1d**         Setal 196 XX         Day 2

| 20µm | EHT = 5.00 kV | Signal A = SE1 | LEO 1530 VP |
| | Mag = 500 X | WD = 10 mm | User Text = |

Figure 1e

Setal 196 XX  Day 13

20µm  EHT = 5.00 kV  Signal A = SE1  LEO 1530 VP
Mag = 500 X  WD = 10 mm  User Text =

EP 3 426 739 B1

Figure 1f

| 20µm | EHT = 5.00 kV | Signal A = SE1 | LEO 1530 VP |
| | Mag = 500 X | WD = 10 mm | User Text = |

EP 3 426 739 B1

**Figure 2b**     Alkyd/Melamine resin     Day 12

20µm

EHT = 5.00 kV     Signal A = SE1     LEO 1530 VP
Mag = 500 X     WD = 10 mm     User Text =

EP 3 426 739 B1

**Figure 2c**                     Alkyd/Melamine resin                     Day 21

| 20µm | EHT = 5.00 kV | Signal A = SE1 | LEO 1530 VP |
| | Mag = 500 X | WD = 10 mm | User Text = |

**Figure 2d**

Blank value (PP-plate without resin)

Day 2

EHT = 5.00 kV    Signal A = SE1    LEO 1530 VP
Mag = 500 X    WD = 10 mm    User Text =

20μm

| 20μm | EHT = 5.00 kV | Signal A = SE1 | LEO 1530 VP |
| | Mag = 500 X | WD = 10 mm | User Text = |

**Figure 2f**    Blank value (PP-plate without resin)    Day 21

| 20µm | EHT = 5.00 kV | Signal A = SE1 | LEO 1530 VP |
| | Mag = 500 X | WD = 10 mm | User Text = |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4039494 A
- WO 201004903 A1
- GB 1599738 A
- EP 5282012 A
- WO 2010040903 A